Europäisches Patentamt

European Patent Office

Office européen des brevets

(11) Publication number: **0 244 363**
**A1**

# EUROPEAN PATENT APPLICATION

(21) Application number: **87810268.0**

(22) Date of filing: **28.04.87**

(51) Int. Cl.⁴: **A 61 K 7/22**

(30) Priority: **02.05.86 US 859113**

(43) Date of publication of application:
**04.11.87 Bulletin 87/45**

(84) Designated Contracting States:
**AT BE CH DE ES FR GB GR IT LI LU NL SE**

(71) Applicant: **WARNER-LAMBERT COMPANY**
**201 Tabor Road**
**Morris Plains New Jersey 07950 (US)**

(72) Inventor: **Pianotti, Roland S.**
**89 Fieldcrest Road**
**Parsippany New Jersey 07054 (US)**

(74) Representative: **Silbiger, Jakob, Dr.**
**c/o CAPSUGEL AG Münchensteinerstrasse 41 Postfach**
**CH-4002 Basel (CH)**

(54) Composition and method to inhibit the growth of organisms by the use of bis-biguanido hexanes and essential oil mixture combinations.

(57) Novel antimicrobial compositions having a synergistic combination of a bis-biguanido hexane compound and a mixture of at least two essential oils and a method for treating teeth to inhibit or prevent dental caries and plaque.

EP 0 244 363 A1

Bundesdruckerei Berlin

## Description

COMPOSITION AND METHOD TO INHIBIT THE GROWTH OF ORGANISMS BY THE USE OF BIS-BIGUANIDO
HEXANES AND ESSENTIAL OIL MIXTURE COMBINATIONS

This invention relates to a novel synergistic antibacterial oral composition to promote oral hygiene which contains in combination a bis-biguanido hexane compound and a mixture of at least two essential oils. Such compositions are useful against the bacteria most commonly associated with the formation of dental plaque and caries such as Streptococcus sanguis and Streptococcus mutans respectively.

Bis-biguanido hexane compounds are well known in the art. These materials possess antibacterial activity and have been used in oral compositions to counter dental plaque and caries formation by bacteria in the oral cavity. The use of these agents at therapeutic levels as the free base or as water soluble salts has resulted in undesirable cosmetic effects. These effects are the brown staining of dental enamel and bitter taste associated with its use. Much effort has been devoted towards overcoming these undersirable effects. Additionally, us of these agents has been reported to cause disquamation of oral epithelial cells and taste aberrations.

U.S. Patent 4,256,731 to Curtis, et al. discloses oral compositions useful for controlling dental plaque and gingivitis and for preventing caries comprising a cationic antimicrobial agent such as bis-biguanide and a combination of antistain agents which reduce the staining effect of the cationic antimicrobial.

U.S. Patent 4,241,049 to Colodney, et al. discloses a dentifrice containing phosphate ion, flavoring oil, 1,6-di-(p-chlorophenyl biguanidohexane) and a water soluble alkaline earth metal salt of a strong acid to stabilize the dentifrice against separation. Calcium and magnesium chlorides are typical stabilizers.

U.S. Patent 4,118,474 to Gaffar, et al. discloses combining the antistain additive phosphonoacetic acid or salts thereof with antibacterial antiplaque agents including bis-biguanido hexanes, such as chlorhexidine and alexidine in an antibacterial oral composition effective to promote oral hygiene.

U.S. Patent 4,080,441 to Gaffar, et al. discloses an antibacterial oral composition effective to promote oral hygiene containing an antibacterial antiplaque agent and an additive which reduces staining of dental surfaces without substantially diminishing the antibacterial and antiplaque activity of the agent. Bis-biguanido hexanes, such as chlorhexidine and alexidine, and quaternary ammonium salts, such as benzethonium chloride and cetyl pyridinium chloride, are typical examples of antibacterial agents. The antistain additive is a bis-(o-carboxyphenyl)ester of a $C_{2-8}$ aliphatic dicarboxylic acid such as bis(o-carboxyphenyl) succinate.

U.S. Patent 3,937,805 to Harrison discloses the preparation of a dentifrice composition containing an insolubilized salt of 1,6-di-(p-chlorophenyl biguanido) hexane antimicrobial agent such as the disarcosinate monomeric salt or monofluorophosphate polymeric salt. The insolubilized salt may be pre-formed or formed in situ in the dentifrice composition. The insolubilized material is antimicrobially effective and provides desirable cosmetic effect in that it does not stain teeth upon repeated use and does not impart an undesirable taste to the oral composition.

U.S. Patent 3,925,543 to Donohue discloses oral products containing bis-biguanido hexane antibacterial agents which are effective in preventing the accumulation of bacterial plaque and which do not stain teeth. Incorporation of a suitable reducing agent, such as ascorbic acid, was found to prevent dental staining while maintaining the activity of the bis-biguanido hexanes in oral products.

The instant invention concerns novel antimicrobial compositions having a synergistic combination of a bis-biguanido hexane compound and a mixture of at least two essential oils selected from the group consisting of thymol, eucalyptol, methyl salicylate and menthol. These antimicrobial compositions reduce dental plaque and caries when incorporated in oral compositions.

As a result of the synergistic combination, the amount of bis-biguanido hexane compound needed to effectively inhibit the growth of organisms such as S. mutans and S. sanguis has been reduced such that the dental staining and bitter taste associated with the use of bis-biguanido hexane compounds has been greatly reduced or eliminated.

The present invention relates to compositions of matter and a method which effectively inhibit or prevent the formation of dental plaque and dental caries. More specifically, it relates to a combination of a bis-biguanido hexane compound and a mixture of at least two essential oils selected from the group consisting of thymol, encalyptol, methyl salicylate and menthol.

A method is provided for treating teeth to inhibit or prevent dental caries and plaque wherein the teeth are contacted with an effective amount of synergistic combination of bis-biguanido hexane and a mixture of at least two essential oils selected from the group consisting of thymol, eucalyptol, methyl salicylate and menthol in an amount sufficient to inhibit growth of S. mutans and S. sanguis present in the oral cavity in general or on teeth in particular.

In carrying out the method of the invention, the combination of a bis-biguanido hexane compound and a mixture of at least two essential oils selected from the group consisting of thymol, eucalyptol, methyl salicylate and menthol will usually be employed in conjunction with a non-toxic carrier such as a mouthwash, or a dentifrice such as a powder, paste, gel, liquid or the like.

Any nontoxic, antibacterial, water-soluble salt of the bis-biguanido hexanes may be employed in the instant invention. The preferred bis-biguanido hexanes are 1, 6-di(p-chlorophenyldiguanido)hexane and 1, 6-bis (2-ethyl-hexyl biguanido)hexane. The preferred acid addition salts are the digluconate, diacetate, dihydrogen halides such as fluoride, chloride and bromide, dimonofluorophosphate, and the like.

In the present invention, the bis-biguanido hexane is present in amounts from about 0.01% to about 10% by weight of the free base form of the compound. Preferably the bis-biguanido hexane is present from about 0.05% to about 1% by weight of the free base form and most preferably from about 0.1% to about 0.5%. The preferred bis-biguanido hexane is 1, 6-di(p-chlorophenyl biguanido)hexane which is also known as chlorhexidine. The preferred salt is the digluconate.

The essential oils of the present invention are selected from the group consisting of thymol, eucalyptol, methyl salicylate and menthol. At least two essential oils must be present. The essential oils are collectively present in an amount from about 0.05% to about 10%, preferably from about 0.1% to about 5% and most preferably about 0.2% to about 4% by weight of the composition. Individually, the essential oils may be present from about 0.025% to about 5% by weight of the composition and preferably from about 0.05% to about 2%. The weight ratio of the least concentrated essential oil to each of the other essential oils is from about 1:5 to about 1:1 and preferably from about 1:3 to about 1:1.

The essence of the present invention is the synergistic effect of inhibiting the growth of bacteria such as S. mutans and S. sanguis which is achieved when a bis-biguanide hexane and a mixture of at least two essential oils selected from the group consisting of thymol, eucalyptol, methyl salicylate and menthol are employed in combination in effective concentrations in the oral cavity. Smaller quantities of each of these materials are required to obtain effective growth inhibition of bacteria such as S. mutans and S. sanguis than if each were employed alone. Sufficient quantities of each material are added to the composition of matter of interest to provide effective amounts of the combination of a bis-biguanido hexane and a mixture of essential oils in the oral cavity. Since lower quantities of bis-biguanido hexane material are used in the synergistic combinations of the present invention, the amount of staining associated with the use of bis-biguanido hexane compounds will be reduced or eliminated.

In one form of the invention, the oral preparation may be a liquid such as a mouthwash or rinse. In such a preparation the vehicle is typically a water-alcohol mixture. Generally the ratio of water to alcohol is in the range of from about 1:1 to about 20:1, preferably about 3:1 to about 20:1 and most preferably about 3:1 to about 10:1 by weight. The total amount of water-alcohol mixture in a mouthwash preparation is typically in the range from about 70% to about 99.9% by weight of the composition. The pH value of such mouthwash preparations is generally from about 4.0 to about 7.0 and preferably from about 5 to about 6.5. A pH below 4 would be irritating to the oral cavity. A pH greater than 7 would result in an unpleasant mouth feel.

Oral liquid preparations may also contain surface active agents in amounts up to about 5% and fluorine-providing compounds in amounts up to about 2% by weight of the preparation.

Surface active agents are organic materials which afford complete dispersion of the preparation throughout the oral cavity. The organic surface active material may be anionic, non-ionic, ampholytic, or cationic. Suitable anionic surfactants are water-soluble salts of higher fatty acid monoglyceride monosulfates, such as the sodium salt of the monosulfated monoglyceride of hydrogenated coconut oil fatty acids; higher alkyl sulfates, such as sodium lauryl sulfate; alkyl aryl sulfonates, such as sodium dodecyl benzene sulfonate; higher alkyl sulfonacetates; higher fatty acid esters of 1,2-dihydroxy propane sulfonates; and substantially saturated higher aliphatic acyl amides of lower aliphatic amino carboxylic acids such as those having 12 to 16 carbons at the fatty acid, alkyl or acyl radicals.

Non-ionic surface active agents include condensates of sorbitan mono-oleate with from 20 to 60 moles of ethylene oxide (e.g., "Tweens" a trademark of ICI United States, Inc.), condensates of ethylene oxide with propylene oxide, condensates of propylene glycol ( "Pluronics" a trademark of BASF-Wyandotte Corp.), and amphoteric agents such as quaternized imidazole derivatives.

Other suitable non-ionic surfactants are the condensation products of an alpha-olefin oxide containing 10 to 20 carbon atoms, a polyhydric alcohol containing 2 to 10 carbons and 2 to 6 hydroxyl groups and either ethylene oxide or a heteric mixture of ethylene oxide and propylene oxide. The resultant surfactants are heteric polymers having a molecular weight in the range of 400 to about 1600 and containing 40% to 80% by weight of ethylene oxide, with a alpha-olefin oxide to polyhydric alcohol mole ratio in the range of about 1:1 to 1:3.

Cationic surface active agents are molecules that carry a positive charge such as cetylpyridinium chloride.

A fluorine providing compound may be present in the oral preparations of this invention. These compounds may be slightly water soluble or may be fully water soluble and are characterized by their ability to release fluroide ions or fluoride containing ions in water. Typical fluorine providing compounds are inorganic fluoride salts such as soluble alkali metal, alkaline earth metal, and heavy metal salts, for example, sodium fluoride, potassium fluoride, ammonium fluoride, cuprous fluoride, zinc fluoride, stannic fluoride, stannous fluoride, barium fluoride, sodium fluorosilicate, ammonium fluorosilicate, sodium fluorozirconate, sodium monofluorophosphate, aluminum mono-and difluorophosphate and fluorinated sodium calcium pyrophosphate.

Alkali metal, tin fluoride and monofluorophosphates such as sodium and stannous fluoride, sodium monofluorophosphate and mixtures thereof are preferred.

The oral preparations of this invention may be substantially solid or pasty in character such as dental cream, toothpaste or a toothpowder. Solid or pasty oral preparations contain polishing materials. Typical polishing materials are abrasive particulate materials having particle sizes of up to about 20 microns. Nonlimiting illustrative examples include: water-insoluble sodium metaphosphate, potassium metaphosphate, tricalcium phosphate, dihydrated calcium phosphate, anhydrous dicalcium phosphate, dicalcium phosphate, calcium pyrophosphate, magnesium orthophosphate, trimagnesium phosphate, calcium carbonate, alumina, aluminum silicate, zirconium silicates, silica, bentonite, and mixtures thereof. Polishing materials are generally present in an amount from about 20% to about 99% by weight of the oral preparation. Preferably, it is present in amounts from about 20% to about 75% in toothpaste, and from about 70% to about 99% in toothpowder.

In clear gels, a polishing agent of colloidal silica and alkali metal aluminosilicate complexes are preferred since they have refractive indicies close to the refractive indicies of gelling agent liquid systems commonly used in dentifrices.

In a oral liquid preparation such as a mouthwash, the fluorine providing compound is generally present in an amount sufficient to release up to about 0.15%, preferably about 0.001% to about 0.1% and most preferably from about 0.001% to about 0.05% fluoride by weight of the preparation.

The oral preparations of the present invention may additionally contain sweetener, flavorants and colorants.

In the instance where auxiliary sweeteners are utilized, the present invention contemplates the inclusion of those sweeteners well known in the art, including both natural and artificial sweeteners. Thus, additional sweeteners may be chosen from the following non-limiting list:

A. Water-soluble sweetening agents such as monosaccharides, disaccharides and polysaccharides such as xylose, ribose, glucose, mannose, galactose, fructose, dextrose, sucrose, maltose, partially hydrolyzed starch, or corn syrup solids and sugar alcohols such as sorbitol, xylitol, mannitol and mixtures thereof.

B. Water-soluble artificial sweeteners such as the soluble saccharin salts, i.e., sodium, or calcium saccharin salts, cyclamate salts, acesulfame-K and the like, and the free acid form of saccharin.

C. Dipeptide based sweeteners such as L-phenylalanine methyl ester and materials described in U.S Patent No. 3,492,131 and the like.

In general, the amount of sweetener will vary with the desired amount of sweeteners selected for a particular oral preparation. This amount will normally be 0.01% to about 40% by weight. The water-soluble sweeteners described in category A above, are preferably used in amounts of from 5% to about 40% by weight, and most preferably from about 10% to about 20% by weight of the final composition. In contrast, the artificial sweeteners described in categories B and C are used in amounts of about 0.005% to about 5.0% and most preferably about 0.05% to about 2.5% by weight of the final composition. These amounts are ordinarily necessary to achieve a desired level of sweetness independent from the flavor level achieved from flavorants.

Suitable flavorings include both natural and artificial flavors, and mints such as peppermint, citrus flavors such as orange and lemon artificial vanilla, cinnamon, various fruit flavors, both individual and mixed, and the like are contemplated. The flavorings are generally utilized in amounts that will vary depending upon the individual flavor, and may, for example, range in amounts of about 0.1% to about 6% by weight of the final composition.

The colorants useful in the present invention, include the pigments which may be incorporated in amounts of up to about 2% by weight of the composition. Also, the colorants may include other dyes suitable for food, drug and cosmetic applications, and known as F.D. & C. dyes and the like. The materials acceptable for the foregoing spectrum of use are preferably water-soluble. Illustrative examples include the indigo dye, known as F.D & C. Blue No. 2, which is the disodium salt of 5,5-indigotindisulfonic acid. Similarly, the dye known as F.D & C. Green No. 1, comprises a triphenylmethane dye and is the monosodium salt of 4-[4-N-ethyl-p-sulfobenzylamino)diphenylmethylene]-[1-(N-ethyl-N-p-sulfoniumbenzyl)-2,5-cyclohexadienimine]. A full recitation of all F.D & C. and D. & C colorants useful in the present invention and their corresponding chemical structures may be found in the Kirk-Othmer Encyclopedia of Chemical Technology, 3rd Edition, in volume 6, at pages 561-595, which text is accordingly incorporated herein by reference.

The present invention is further illustrated by the following examples. All parts and percentages in the examples and throughout the specification and claims are by total weight unless otherwise indicated.

The following definitions and procedures are utilized throughout the examples.

## MINIMAL GROWTH INHIBITORY CONCENTRATION

The minimal inhibitory concentration (MIC) of the test compound is the minimum concentration of compound required to inhibit growth of the test organism. The MIC is determined by serially diluting the test solution through tubes of sterile broth. The tubes are inoculated and incubated at 37°C for 48 hours. At the end of the incubation time, growth is checked macroscopically. The lowest concentration of test compound to inhibit growth is the MIC.

## CHECKERBOARD TITRATION

This technique is used to assess antibacterial activity of combinations. The 'checkerboard' is the pattern found by multiple combinations of two antimicrobial agents in concentrations equal to, above, and below their minimal inhibitory concentration for the organism being tested. The checkerboard consists of columns which contain the same amount of one antimicrobial which is diluted along the x-axis and rows which each contain the same amount of the antimicrobial (diluted along the y-axis). Thus each tube is a unique combination of the two agents.

A method of quantitating synergy is the 'fractional inhibitory concentration' (FIC) index. For each row, the FIC index is calculated from the lowest concentration of agent necessary to inhibit growth. The FIC of

4

each agent is derived by dividing the concentration of the agent present in that tube by the minimal inhibitory concentration of the organism to that agent alone. The FIC index is then the sum of these values for both agents at that point.

Fractional Inhibitory Concentration Index is used as a measure of synergy. When the FIC index is equal to or less than 0.5, the combination is synergistic. When the FIC index is greater than 0.5 but less than 2.0 the combination is additive. When the FIC index is greater than 2.0 the combination is antagonistic. The FIC index is calculated as follows:

$$\text{FIC index} = \frac{\text{MIC(A) in the presence of B}}{\text{MICA}} + \frac{\text{MIC(B) in the presence of A}}{\text{MICB}}$$

where:

MIC(A) is the concentration of antimicrobial A in a tube which is the lowest inhibitory concentration in its row from the checkerboard titration. MICA is the MIC of the organism to antimicrobial A alone. FICA is the fractional inhibitory concentration of antimicrobial A. MIC(B) MICB, and FICB are defined in the same fashion for antimicrobial B.

Vehicle:

1.5 grams of benzoic acid is dissolved in one liter of 28.5% enthanol in water (v/v).

EXAMPLE 1

Inventive Experiments 1, 2, 3 and 4

This example illustrates the Minimum Inhibitory Concentration (MIC) and Fractional Inhibitory Concentration (FIC) index of a mixture of essential oils, and chlorhexidine alone and in combination for the bacterium Streptococcus mutans ATCC 25175. The experiment was repeated four times.

**MIC in w/w%**

| Experiment | 1 | 2 | 3 | 4 |
|---|---|---|---|---|
| Essential Oil mixture (1) | 0.318 | 0.318 | 0.318 | 0.159 |
| Chlorhexidine (2) | $2.2 \times 10^{-5}$ | $2.2 \times 10^{-5}$ | $2.8 \times 10^{-5}$ | $2.25 \times 10^{-5}$ |
| Essential Oil Mixture in Chlorhexidine (3) | 0.0381 | 0.0381 | 0.0381 | 0.0198 |
| Chlorhexidine in Essential Oil Mixture (3) | $5.5 \times 10^{-6}$ | $5.5 \times 10^{-6}$ | $1.7 \times 10^{-6}$ | $2.8 \times 10^{-6}$ |
| FIC Index | 0.37 | 0.37 | 0.18 | 0.25 |

(1)   Essential oil mixture of Thymol, eucalyptol, methyl salicylate and menthol.

(2)   Free base concentration calculated from the chlorhexidine digluconate salt (MW=897.5)

(3)   Calculated from the checkerboard citration method.

All four experiments demonstrate synergy between chlorhexidine and the mixture of essential oils as determined by an FIC index of less than or equal to 0.5.

EXAMPLE 2

Non-Inventive Experiments A, B, C, and D

This example illustrates the Minimum Inhibitory Concentration (MIC) and Fractional Inhibitory Concentration (FIC) index of Chlorhexidine, and individual essential oils alone and in combinations of individual essential oils with chlorhexidine for the bacterium Streptococcus mutans ATCC 25175.

| | TEST Compound | MIC (micro g/ml) | FIC Index |
|---|---|---|---|
| A. | Thymol | 80 | |
| | Thymol in Chlorhexidine(1) | 40 | |
| | Chlorhexidine | 0.50 | |
| | Chlorhexidine in Thymol(1) | 0.250 | 1.00 |
| B. | Eucalyptol | 58 | |
| | Eucalyptol in Chlorhexidine(1) | 58 | |
| | Chlorhexidine | 0.250 | |
| | Chlorhexidine in Eucalyptol(1) | 0.062 | 1.248 |
| C. | Methyl Salicylate | 35 | |
| | Methyl Salicylate in Chlorhexidine(1) | 35 | |
| | Chlorhexidine | .250 | |
| | Chlorhexidine in Methyl Salicylate(1) | 0.250 | 2.00 |
| D. | Menthol | 30 | |
| | Menthol in Chlorhexidine(1) | 10 | |
| | Chlorhexidine | 0.250 | |
| | Chlorhexidine in menthol(1) | 0.062 | 0.581 |

(1) Calculated from the checkerboard titration method.

None of the tested combinations demonstrates synergy as determined by the FIC index. All combinations have an FIC index greater than 0.5.

EXAMPLE 3

Inventive experiments 5, 6 and 7

This example illustrates the Minimum Inhibitory Concentration (MIC) and Fractional Inhibitory Concentration (FIC) index of a mixture of essential oils, and chlorhexidine alone and in combination for the bacteria Streptococcus sanguis ATCC 19558, Actinomyces viscosus T14V and Candida albicans ATCC 18804.

| Experiment | 5 | 6 MIC (w/w%) | 7 |
|---|---|---|---|
| Organism | S. sanguis | A. viscosus | C. Albicans |
| Essential Oil Mixture (1) | 0.318 | 0.159 | 0.318 |
| Chlorhexidine (2) | $3.4 \times 10^{-4}$ | $4.4 \times 10^{-5}$ | $3.5 \times 10^{-4}$ |
| Essential Oil Mixture in Chlorhexidine | 0.079 | 0.039 | 0.079 |
| Chlorhexidine in Essential Oil Mixture | $5.6 \times 10^{-6}$ | $2.2 \times 10^{-5}$ | $8.8 \times 10^{-5}$ |
| FIC Index | 0.264 | 0.745 | 0.500 |

(1)   Essential oil mixture of Thymol, Eucalyptol, methyl salicylate and menthol.

(2)   Free base concentration calculated from chlorhexidine digluconate salt (MW = 897.5).

(3)   Calculated from the checkerboard titration method.

The tested combinations show synergy for S. sanguis and C. albicans and a strong additive effect for A. viscosus as determined by the FIC index.

EXAMPLE 4
This example demonstrates a mouthrinse of the present invention prepared by mixing together the following ingredients:

| Ingredient | Percent by Weight |
|---|---|
| Ethanol | 25.0 |
| Nonionic Surfactant | 2.0 |
| Benzoic Acid | 0.15 |
| Chlorhexidine digluconate (20% w/v solution) | 0.05 |
| Water, Potable q.s. to | 1.00 liter |
| Color | 0.2 |
| Sweetener | 0.02 |
| Essential Oils: | 2.40 |

| | |
|---|---|
| Thymol | 0.6 |
| Eucalyptol | 0.6 |
| Methyl salicylate | 0.6 |
| Menthol | 0.6 |

Sodium hydroxide adjust to pH 4.2

The final pH of the above mouthrinse is adjusted to between 4.1 and 4.3 with sodium hydroxide. This mouthrinse shows improved antimicrobial activity.

This invention being thus described, it will be obvious that the same may be varied in many ways. Such variations are not be regarded as a departure from the spirit and scope of the invention and all such modifications are intended to be included within the scope of the following claims.

**Claims**

1. An antibacterial oral composition comprising an oral vehicle, an antibacterial bis-biguanido hexane compound and a mixture of at least two essential oils selected from the group consisting of thymol, eucalyptol, methyl salicylate and menthol.

2. The oral composition of Claim 1 wherein the antibacterial bis-biguanido hexane compound is a pharmaceutically acceptable water soluble salt of a compound selected from the group consisting of chlorhexidine and alexidine.

3. The oral composition of the claims 1 or 2, wherein the antibacterial bis-biguanido hexane compound is present in an amount from about 0.01% to about 10% by weight of the composition based on the free base form of said compound.

4. The oral composition according to anyone of the claims 1 to 3, wherein the mixture of essential oils is present in an amount from about 0.05% to about 10% by weight of the composition.

5. The oral composition according to anyone of the claims 1 to 4, wherein the oral vehicle comprises a liquid vehicle having a pH value of about 4.0 to about 7.0.

6. The oral composition according to anyone of the claims 1 to 5, wherein the oral vehicle is an aqueous-alcohol combination and said antibacterial oral composition is a mouthwash having a pH value of about 4.0 to about 7.0.

7. The mouthwash composition of Claim 6 wherein the bis-biguanido hexane compound is a water-soluble pharmaceutically acceptable chlorhexidine salt present in an amount from about 0.01% to about 10% based on its free base weight and the mixture of essential oils are present in an amount from about 0.05% to about 10% all percents are by weight of the total composition.

8. A method of improving oral hygiene comprising applying to the oral cavity an effective amount of an antibacterial oral composition as defined in Claim 1.

9. The method of Claim 8 wherein said antibacterial oral combination is contacted with an organism selected from the group consisting of Streptococcus mutans, Streptococcus sanguis, and mixtures thereof.

8

European. Patent Office

**PARTIAL EUROPEAN SEARCH REPORT**
which under Rule 45 of the European Patent Convention shall be considered, for the purposes of subsequent proceedings, as the European search report

Application number

EP 87 81 0268

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 365 030 (MERCK) <br><br> * Claims; page 1, lines 46-72; example 2 * <br><br> --- | 1-7 | A 61 K  7/22 |
| X | DE-A-1 964 196 (MERCK) <br><br> * Claims 1,3,7,8; example 3 * <br><br> --- | 1-7 | |
| X | FR-A-2 440 189 (UNILEVER) <br><br> * Claims 1,8,9,11,12; page 6, lines 5-25; examples * <br><br> ---------------- | 1-7 | |

TECHNICAL FIELDS SEARCHED (Int. Cl.4)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely: 8,9
Claims not searched:
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 15-07-1987 | WILLEKENS |

EPO Form 1505.1 .03.82